# EUROPEAN PATENT APPLICATION

(11) **EP 4 183 442 A1**
(43) Date of publication of application: **24.05.2023**
(21) Application number: 21864681.8
(22) Date of filing: 02.09.2021
(51) Int. Cl.: A61M 37/00, A61M 5/46

(54) **TATTOO DEVICE**

(30) Priority: 07.09.2020 KR 20200114147; 23.12.2020 KR 20200181523; 18.08.2021 KR 20210108911
(71) Applicant: Kyon, Byongdok, Pyeongtaek-si, Gyeonggi-do 17917 (KR); Lee, Soonmi, Yongin-si, Gyeonggi-do 16903 (KR)
(72) Inventor: Kyon, Byongdok, Pyeongtaek-si, Gyeonggi-do 17917 (KR); Lee, Soonmi, Yongin-si, Gyeonggi-do 16903 (KR)
(74) Representative: M. Zardi & Co S.A.
(86) International application number: PCT/KR2021/011855
(87) International publication number: WO 2022/050722

(57) **Abstract**

The present invention relates to a tattoo device. The tattoo device includes: a tattoo device body including a setting unit configured to set a needle draw distance, a needle draw speed, and the amount of pigment to be injected, and a tattoo control unit configured to control tattoo pigment to be accurately injected into a target skin layer by individually controlling the draw speed of a needle and a needle pushing force according to the needle draw distance, the needle draw speed, and the amount of pigment to be injected set by the setting unit; and a handpiece coupled to the tattoo device body, and including a needle drive unit that controls the driving of the needle mounted in a needle unit by using a solenoid under the control of the tattoo control unit.

## Description

### Technical Field

The present invention relates to a tattoo device, and more particularly to a tattoo device that injects pigment for tattooing into an accurate scalp layer by automatically adjusting the depth at which a needle for injecting pigment enters the skin, that allows more controlled and precise dots to be marked by individually controlling the speed and force (torque) between the up and down movements of the needle while avoiding the method of a convention tattoo device in which when the speed of the up and down movement of a needle increases, force becomes stronger, and that can prevent pigment from spreading by controlling color intensity in such a way as to control the amount of pigment to be injected through the adjustment of the number of penetrations of a needle per dot.

### Background Art

Tattooing refers to a procedure in which pigment is injected by marking fine dots in areas where hair is lacking or has disappeared. Like hair transplantation, it has the advantage of being able to reduce the time and cost of the procedure while highlighting a visual effect.

Tattooing is a technique in which a practitioner applies pigment to a considerably fast reciprocating needle using the wrist and then marks dots on the skin.

It is basically necessary to mark dots ranging from thousands of dots to tens of thousands of dots. This requires a high level of concentration, and tattooing takes three to eight hours. Furthermore, the dots formed to be shallow because the depth cannot be adjusted disappear quickly, so that it is necessary to repeat tattooing a plurality of times rather than performing tattooing once, which causes physical fatigue.

In particular, a practitioner's eyes need to always view the tip of a needle, and the wrist needs to move a number of times equal to the number of dots, resulting in severe physical fatigue. Accordingly, there is a demand for a tattoo device that can perform tattooing accurately while reducing stress on the wrist and can perform tattooing within a short time.

When a tattoo is marked using black pigment, the black pigment will turn blue over time and then turn green over time. In order to minimize this discoloration, it is very important to control the depth of a needle for injecting pigment, the amount of pigment, and even injection into the exact upper dermis layer of the scalp without damaging the hair follicle.

In particular, when dots are marked on the skin by using pigment, it is important to mark dots at predetermined intervals and depths. In this case, when the predetermined intervals, the predetermined depths or the difference between the times when the dots are marked are different, the size of the dots is different or a color is different only in a specific area. This occurs when the time for which the hand is moved is different or the pressure applied to the scalp is different in the case where a practitioner performs tattooing by using the hand.

Conventional tattoo devices mainly use a rotary motor method or a coil method.

The rotary motor method has disadvantages in that it is difficult to exert force at low speed (rpm), the vertical movement distance of a needle is fixed, and the acceleration/deceleration period is long in the vertical movement of the needle, thus causing damage to the skin. In this case, in the rotary motor method, the needle continues to move up and down, so that the drawing distance of the needle cannot be adjusted or set.

In addition, the coil method has the advantage of being able to adjust the vertical movement distance of a needle, but has a disadvantage in that noise and vibration are large.

Accordingly, in recent years, various tattoo equipment and methods have been proposed to mitigate these problems and allow tattoos to be applied evenly onto the correct skin layer.

Conventionally proposed tattoo devices are disclosed in <patent document 1> to <patent document 4> below.

The prior art disclosed in <patent document 1> includes a drive member built into a main body and provided with a driving shaft that is linearly moved by an eccentric rotating body, a needle slider configured to operate in conjunction with the driving shaft, and a needle guide provided with a needle member having a needle and a needle guide groove coupled to one side of the main body and configured to allow the needle to move in a straight line. Through this configuration, there is provided a skin treatment device that can treat the wrinkles, dead skin cells, scars, melasma, stretch marks, hair loss, etc. of the human skin using needles or engrave pictures or characters using pigment.

In addition, the prior art disclosed in <patent document 2> can be used for needling the skin of a subject, and a drug applicator device can be used to apply a drug to the skin of a subject. For example, the needling device can be applied to the skin of a subject for hair growth applications, or can be used for wrinkle, scar, or tattoo removal. The drug applicator device provides a needling device and a drug applicator that can be used for multiple drug application purposes, such as the application of a hair growth compound to the skin of a subject.

Furthermore, the prior art disclosed in <patent document 3> provides a semi-permanent tattooing method for eyebrow tattoos or like in which the tattooed lines of the eyebrows or hairline are raised and look like real hair, so they are natural like their own eyebrows or hair, and the pigment penetrates only to the upper dermal layer, so that there are almost no side effects and so that they disappear naturally after about 2 years and thus a user can tattoo with a new taste, with the result that there is no hassle of tattoo removal surgery.

Moreover, the prior art disclosed in <patent document 4> includes a body configured to generate its own weight, an actuating member configured to press an actuating pin and a spring, a support cover configured to support a fixing unit formed on the actuating member, and a grip cover configured to assemble and fix the actuating member, assembled with the support cover in an integrated manner, to the body. Through this configuration, there is provided a semi-permanent makeup device that minimizes the burden on the wrist of a practitioner and, at the same time, reduces the probability of failure of a procedure and promotes safety from the point of view of a subject.
(Patent document 1) Korean Patent No. 10-1131577 (registered on March 22, 2012) (Skin Treatment Device)
(Patent document 2) Korean Patent Application Publication No. 10-2018-0084759 (published on July 25, 2018) (Needling Device and Drug Applicator)
(Patent document 3) Korean Patent No. 10-1565340 (registered on October 28, 2015) (Semi-Permanent Tattooing Method for Eyebrows, Etc.)
(Patent document 4) Korean Patent Application Publication No. 10-2018-0073325 (published on July 2, 2018) (Semi-Permanent Tattooing and Cosmetic Device with Elasticity Adjustment Function Using Grip Cover and Self-Weight)

### Disclosure

### Technical Problem

However, in the conventional tattoo devices and the prior art described above, in the case where, when a practitioner performs tattooing using the hand, the movement time of the hand varies or the pressure applied to the skin varies, a pigment injection layer varies, so that the color of a specific location becomes darker or pigment spreads. The conventional tattoo devices and prior art cannot compensate for the difference in hand movement time or pressure. Accordingly, the conventional tattoo devices and the prior art are disadvantageous in that the spreading of a color or easy discoloration occurs after tattooing.

In addition, the conventional tattoo devices and the prior art are disadvantageous in that a high degree of concentration and a long tattooing time are required, the eyes of a practitioner need always view the tip of a needle, and the wrist need to move a number of times equal to the number of dots, resulting in severe physical fatigue.

Therefore, the present invention has been proposed to overcome all the problems occurring in the prior arts described above, and an object of the present invention is to provide a tattoo device that is capable of injecting pigment for tattooing into an accurate scalp layer by fully automatically adjusting the depth at which a needle for injecting pigment enters the skin.

Another object of the present invention is to provide a tattoo device that is capable of adjusting the amount of pigment to be injected by setting the number of penetrations of a needle to 1 to 5 when marking a single dot. That is, there is provided a tattoo device that, when a dot is marked at one location, injects a predetermined amount of pigment by hitting the same spot up to one to five times to mark the dot.

Another object of the present invention is to provide a tattoo device that is capable of injecting pigment into a precise and constant scalp layer by measuring the pressure with the skin and automatically adjusting the draw distance of a needle in conjunction with the measured pressure.

Still another object of the present invention is to provide a tattoo device that detects the amount of movement of a handpiece and automatically stops the drawing of a needle when the movement time of the hand is out of the range of the set movement time, thereby preventing staining or a color change at a specific location.

Still another object of the present invention is to provide a tattoo device that individually controls the speed (rpm) and force (torque) of the needle to output set power/torque regardless of the speed when the needle is moved up and down, so that sufficient force is transmitted even at low speed so that there is no difficulty in penetrating the skin, unlike in a rotary motor.

In other words, the matrix of points is expressed as lines as the distance between dots is closer. The present invention provides a tattoo device that expresses dots as dots, lowers the speed of vertical movement, and facilitates penetration into the skin even at a low speed.

### Technical Solution

In order to accomplish the above objects, the present invention provides a "tattoo device" including:
a tattoo device body including a setting unit configured to set a needle draw distance, a needle draw speed, and the amount of pigment to be injected, and a tattoo control unit configured to control tattoo pigment to be accurately injected into a target skin layer by individually controlling the draw speed of a needle and a needle pushing force according to the needle draw distance, the needle draw speed, and the amount of pigment to be injected set by the setting unit; and
a handpiece coupled to the tattoo device body, and including a needle drive unit that controls the driving of the needle mounted in a needle unit by using a solenoid under the control of the tattoo control unit.

In the above-described tattoo device, the handpiece may further include:
the needle unit equipped with the needle;
a needle guide configured to guide the needle through the drawing thereof, to prevent hair from coming into direct contact with the needle during scalp tattooing, and to fix the skin, which may shake when the needle penetrates the skin, by applying pressure after coming into contact with the skin so that the needle is made to accurately penetrate a set skin layer;
a movement speed detection unit configured to detect a movement speed of the handpiece; and
a pressure detection unit configured to detect a pressure between the skin and the needle unit;
wherein the tattoo control unit controls the operations of the tattoo device according to the detected movement speed and the detected pressure value.

In the above-described tattoo device, the tattoo control unit may control the draw speed of the needle by adjusting the interval between the operations of the solenoid.

In the above-described tattoo device, the tattoo control unit may control a burst mode by adjusting the times of the ON/OFF operations of the solenoid.

In the above-described tattoo device, the tattoo control unit may adjust the depth at which the needle penetrates the skin by adjusting the needle pushing force according to the skin.

In the above-described tattoo device, the tattoo control unit may include a needle draw speed setting unit configured to set a needle draw speed based on the needle draw speed set by the setting unit, a needle draw distance setting unit configured to set a needle draw distance based on the needle draw distance set by the setting unit, and a pigment injection amount setting unit configured to set an amount of pigment to be injected based on the amount of pigment to be injected set by the setting unit.

In the above-described tattoo device, the tattoo control unit may further include a needle speed control unit configured to control a needle driving speed according to the set needle draw speed and to control ON/OFF operations of the solenoid based on a detected handpiece movement speed or the set needle draw speed, a needle draw distance control unit configured to variably control a needle draw distance in real time according to the set needle draw distance and a detected pressure value, and a needle torque control unit configured to control a needle pushing torque according to the set amount of pigment to be injected, thereby preventing the pigment from spreading.

In the above-described tattoo device, the needle speed control unit may control the number of dots at one point by adjusting the interval between the ON/OFF operations of the solenoid for drawing the needle, and may control a burst mode by adjusting the number of the ON/ OFF operations of the solenoid per cycle.

In the above-described tattoo device, the needle draw distance control unit may control the draw distance of the needle by adjusting the ON driving time of the solenoid.

### Advantageous Effects

According to the present invention, there is an effect in which a needle driving speed and a needle pushing force are individually controlled, so that the force required to penetrate the skin is transmitted through the control of torque, which is an instantaneous force, even in a situation in which the speed is low in order to allow pigment to effectively penetrate the skin, thereby always implementing a uniform tattoo regardless of the needle driving speed.

According to the present invention, there is an advantage in that pigment for tattooing may be injected into an accurate skin layer by automatically adjusting the draw distance of the needle for the injection of the pigment.

In addition, according to the present invention, there is an advantage in that the pressure with the skin is measured and the draw distance of the needle is automatically adjusted in conjunction with the measured pressure, so that pigment can be injected into an accurate and constant scalp layer, thereby preventing discoloration.

In addition, according to the present invention, there is an advantage in that the amount of movement of the handpiece including the needle is detected and the needle draw speed is automatically reduced when the movement time of the hand is out of the range of the set movement time, so that staining or a change in the density of dots at a specific location can be prevented, thereby promoting uniform tattooing.

### Description of Drawings

FIG. 1 is a diagram of the overall configuration of a tattoo device according to the present invention;
FIG. 2 is a block diagram showing a specific embodiment of the tattoo control unit of FIG. 1;
FIG. 3 is a diagram illustrating the adjustment of the driving speed of a needle per time in the present invention;
FIG. 4 is a diagram showing an example of solenoid driving timing for the control of a burst mode in the present invention;
FIG. 5 is a diagram illustrating a needle pushing force in the present invention;
FIG. 6 is a view illustrating the adjustment of the draw distance of the needle through the ON drive time control of a solenoid in the present invention;
FIG. 7 is a view of a tattoo device according to the present invention; and
FIG. 8 is a perspective view of a handpiece according to the present invention.

### Best Mode

A tattoo device according to a preferred embodiment of the present invention will be described in detail below with reference to the accompanying drawings.

The terms or words used in the present invention described below should not be construed as being limited to common or dictionary meanings. The terms or words should be interpreted as having meanings and concepts consistent with the technical spirit of the present invention based on the principle that an inventor can appropriately define the concepts of terms in order to explain his or her invention in the best way.

Therefore, the embodiments described in the present specification and the configurations shown in the drawings are only preferred embodiments of the present invention, and do not represent the overall technical spirit of the present invention. It should be understood that there may be various equivalents and variations that can be substituted for the above embodiments and configurations at the time when the present application is filed.

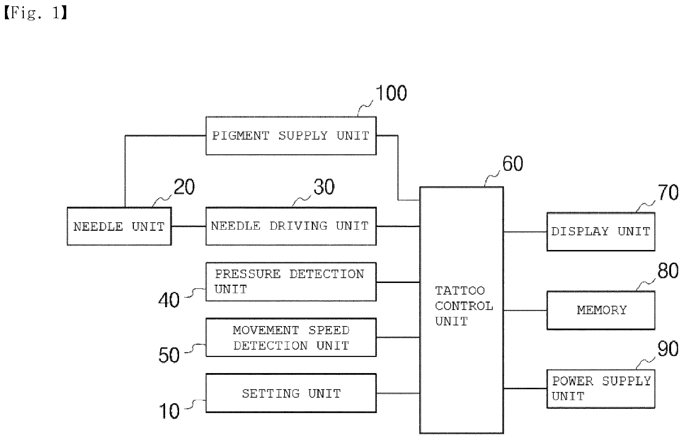

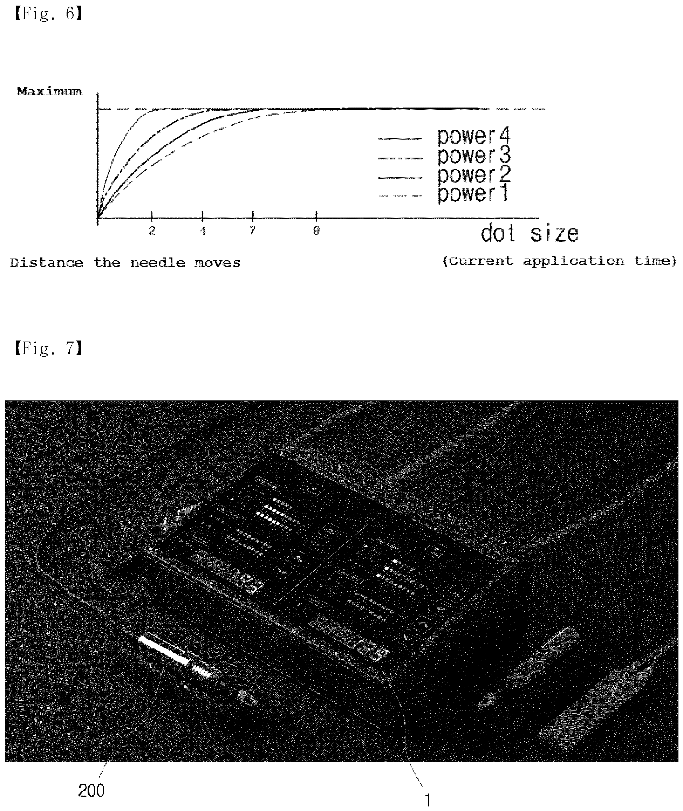

FIG. 1 is a diagram of the overall configuration of a tattoo device according to a preferred embodiment of the present invention, and FIG. 7 is a perspective view thereof. The tattoo device includes: a tattoo device body 1 including a setting unit 10, a tattoo control unit 60, a display unit 70, memory 80, and a power supply unit 90; and a handpiece 200 including a needle unit 20, a needle driving unit 30, a pressure detection unit 40, a movement speed detection unit 50, and a pigment supply unit 100.

That is, the tattoo device according to the present invention basically includes the tattoo device body 1, and the handpiece 200 connected to the tattoo device body 1 so that a practitioner applies a tattoo.

The tattoo device body 1 includes the setting unit 10 configured to set a needle draw distance, a needle draw speed, and the amount of pigment to be injected, and the tattoo control unit 60 configured to control tattoo pigment to be accurately injected into a target skin layer (the skin or the scalp) by individually controlling the draw speed of the needle and a needle pushing force according to the needle draw distance, the needle draw speed, and the amount of pigment to be injected set by the setting unit 10.

In this case, it is preferable that a practitioner, who is an expert, set the needle draw distance after inspecting the condition of the skin.

The tattoo control unit 60 controls a burst mode by adjusting the times of the ON/OFF operations of a solenoid, which is a needle driving means provided in the handpiece 200. In this case, the burst mode is a mode which determines how many strokes are applied at one stroke location. For example, in the case of a 2-stroke burst mode, it is a mode in which two strokes are applied at one stroke location, and in the case of the 4-stroke burst mode, it is a mode in which four strokes are applied at one stroke location.

In addition, the tattoo control unit 60 may control the depth at which a needle penetrates the skin by adjusting a needle pushing force according to the skin.

The tattoo control unit 60 includes a needle draw speed setting unit 61 configured to set a needle draw speed based on the needle draw speed set by the setting unit 10, a needle draw distance setting unit 62 configured to set a needle draw distance based on the needle draw distance set by the setting unit 10, and a pigment injection amount setting unit 63 configured to set the amount of pigment to be injected based on the amount of pigment to be injected set by the setting unit 10.

In addition, the tattoo control unit 60 may further include a needle speed control unit 66 configured to control a needle driving speed according to the set needle draw speed and to control the ON/OFF operations of the solenoid based on a detected handpiece movement speed, a needle draw distance control unit 68 configured to variably control a needle draw distance in real time according to the set needle draw distance and a detected pressure value, and a needle torque control unit 67 configured to control a needle pushing torque (force) according to the set amount of pigment to be injected, thereby preventing the pigment from spreading.

The needle speed control unit 66 adjusts the number of strokes at one location by adjusting the interval between the ON/OFF operations of the solenoid for drawing the needle, and also adjusts the burst mode by adjusting the number of ON/OFF operations of the solenoid per cycle.

The needle draw distance control unit 68 adjusts the draw distance of the needle by adjusting the ON driving time of the solenoid.

The display unit 70 serves to display setting values set by a practitioner and to show information such as the tattoo time and the number of punches (strokes). The memory 80 serves to store information such as the needle draw speed, the needle draw distance, and the amount of pigment to be injected set by a practitioner and to store control values for the control of the needle draw speed and the needle draw distance and control values for the amount of pigment to be injected.

The power supply unit 90 is a part that supplies driving power to the tattoo device, and may use a built-in battery or an external commercial AC power source. When an external commercial AC power source is used, a built-in battery may be used as a secondary battery and charged using the external commercial AC power source.

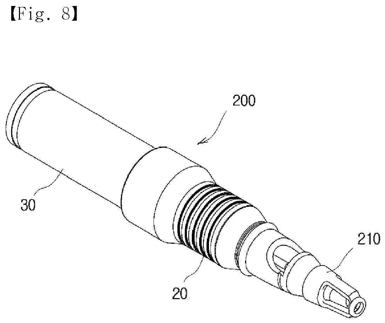

As shown in FIG. 8, the handpiece 200 is coupled to the tattoo device body 1, and includes a needle drive unit 30 configured to control the driving of the needle mounted on a needle unit 20 by using the solenoid under the control of the tattoo control unit 60, the needle unit 20 equipped with the needle, and a needle guide 210 configured to guide the needle through the drawing thereof.

In this case, the needle guide 210 serves to guide the needle through the drawing thereof, to prevent hair from coming into direct contact with the needle during scalp tattooing, and to fix the skin, which may shake when the needle penetrates the skin, by applying pressure after coming into contact with the skin so that the needle is made to accurately penetrate a set skin layer.

The needle unit 20 is configured in such a manner that the needle configured to inject pigment for tattooing into the scalp is contained in a probe that comes into contact with the skin.

The handpiece 200 may further include the movement speed detection unit 50 configured to detect the movement speed of the handpiece, and the pressure detection unit 40 configured to detect the pressure between the skin and the needle unit 20.

The movement speed detection unit 50 may use an acceleration sensor or the like to detect the movement speed, and the pressure detection unit 40 may use a pressure sensor and is preferably provided at an end of the probe.

In addition, the needle driving unit 30 includes the solenoid for driving the needle.

The tattoo control unit 60 controls the operation of the tattoo device according to a detected movement speed and a detected pressure value, and controls the draw speed of the needle by adjusting the operation interval of the solenoid.

That is, when the handpiece 200 enters a tattooing mode, the needle is repeatedly moved as current flows through a solenoid coil and is cut off, and thus the needle comes into contact with the skin, thereby performing tattooing. When the current flows through the solenoid coil, an attractive force is instantaneously applied to a plunger by an electromagnetic force, so that the plunger moves toward the needle assembly side instantaneously. Accordingly, a plunger actuating pin overcomes the elastic force of a silicon spring and forcibly pushes a needle-integrated needle pin, so that the needle is moved forward. In addition, when the current is cut off in the solenoid coil, the needle-integrated needle pin reversely pushes the plunger actuating pin by the return force of the forcibly stretched silicon spring, so that the plunger is also returned to its original position, thereby implementing the repetitive movement of the needle.

To this end, the present invention uses a silicon spring that is sensitive to external forces and has lower elasticity and repulsive coefficient than conventional metal springs. When the needle assembly is assembled to a connector, the plunger actuating pin and the needle-integrated needle pin are brought into close contact with each other inside a core by the elastic force of the slightly extended silicon spring.

That is, according to the present invention, when a silicon spring having lower elasticity and repulsive coefficient than a spring made of metal and being sensitive to external force is used and the plunger is moved forward by the electromagnetic force of the coil, force is transmitted to the needle-integrated needle pin through the plunger actuating pin. In this case, the plunger actuating pin and the needle-integrated needle pin are brought into contact with each other and maintained in close contact with each other by the elastic force of the silicon spring, so that force can be immediately and rapidly transferred to the needle.

Using the tattoo device configured as described above, a natural hairstyle is implemented by injecting pigment as if dots are applied to an area where hair is lacking or has disappeared, and a desired tattoo is implemented on the skin.

The detailed description of the operation of the tattoo device according to the present invention configured as described above is given as follows:
First, a practitioner supplies power through the power supply unit 90 in order to use the tattoo device. When driving power is supplied by the power supply unit 90, the tattoo control unit 60 performs initialization and waits for input values set by the setting unit 10. In this case, when the user performs operation through the input unit without separately setting a needle draw speed, a needle draw distance, and the amount of pigment to be injected, the tattoo control unit 60 fetches information such as a previously used needle draw speed, a previously used needle draw distance, and the previously used amount of pigment to be injected stored in the memory 80 and then controls the operation of the tattoo device based thereon.

Unlike this, when a practitioner sets a needle draw speed, a needle draw distance, and the amount of pigment to be injected through the setting unit 10, the operation of the tattoo device is controlled based on these values. In this case, the user (the practitioner) sets the needle draw distance and the amount of pigment to be injected after inspecting the skin condition of a tattooing target person. The skin conditions of the human bodies may be different. The skin condition of some may be hard, while the skin condition of others may be relatively soft. Tattoos are closely related to the depths of the needle and the amounts of pigment. In particular, discoloration or staining may be prevented and also a natural tattoo or hairstyle may be generated only when pigment is precisely injected into the thin layer of the skin. Accordingly, it will be very important to determine the needle draw distance and the amount of pigment to be injected by checking the skin condition of a tattooing target person. Therefore, it is desirable that an expert (a practitioner) with a lot of experience and know-how perform tattooing.

It is also preferable that the needle draw speed be set by an expert after inspecting the skin condition, that is, long hair, bald hair, etc. in the case of scalp tattooing.

When the practitioner sets the needle draw distance, the speed, and the amount of pigment to be injected through the setting unit 10, the tattoo control unit 60 controls the operation of the tattoo device based on these setting values.

That is, the tattoo control unit 60 extracts control values from the memory 80 based on the needle draw distance, the draw speed, and the amount pigment of to be injected set by the setting unit 10 and controls the main operation of the scalp tattoo device. The tattoo control unit 60 additionally controls the operation of the tattoo device by referring to the movement speed and a detected pressure value and individually controlling the draw speed and the force.

For example, the needle draw distance setting unit 62 of the tattoo control unit 60 extracts a needle draw distance control value from the memory 80 based on the needle draw distance set by the setting unit 10, and sets the needle draw distance.

Using the needle draw distance control value set in this way, the needle draw distance control unit 68 adjusts the needle draw distance by adjusting the ON driving time of the solenoid provided in the needle driving unit 30 of the handpiece 200.

As shown in FIG. 6, the final movement distance of the needle, that is, the draw distance, is adjusted by adjusting the time for which the driving current is applied to the solenoid.

In FIG. 6, the supply time of power, which is the driving current, has a relationship of power 1 < power 2 < power 3 < power 4, which are the ON driving times of the solenoid.

That is, when the driving current is applied for the time of power 1, the needle draw distance becomes shortest. When the driving current is applied for the time of power2, the needle draw distance increases to be longer than in the case of the time of power 1. When the driving current is applied for the time of power 3, the needle draw distance increases to be longer than in the case of the time of power 2. When the driving current is applied for the time of power 4, the needle draw distance increases to be longer than in the case of the time of power 3.

In other words, in the case where the time taken for the needle to be drawn to the maximum value at the same voltage is 1 (power 4), when the current application time is set to be less than 1, the moving distance of the needle is also reduced. As described above, the needle draw distance is adjusted by adjusting the driving ON time of the solenoid.

In addition, the needle draw speed setting unit 61 extracts a needle draw speed control value from the memory 80 based on the needle draw speed (rpm) set by the setting unit 10 and then sets the needle draw speed.

The needle speed control unit 66 adjusts the number of strokes, that is, the needle speed (rpm), at one location by adjusting the interval between the ON/OFF operations of the solenoid of the needle driving unit 30 for drawing the needle in response to the setting of the needle draw speed by the needle draw speed setting unit 61.

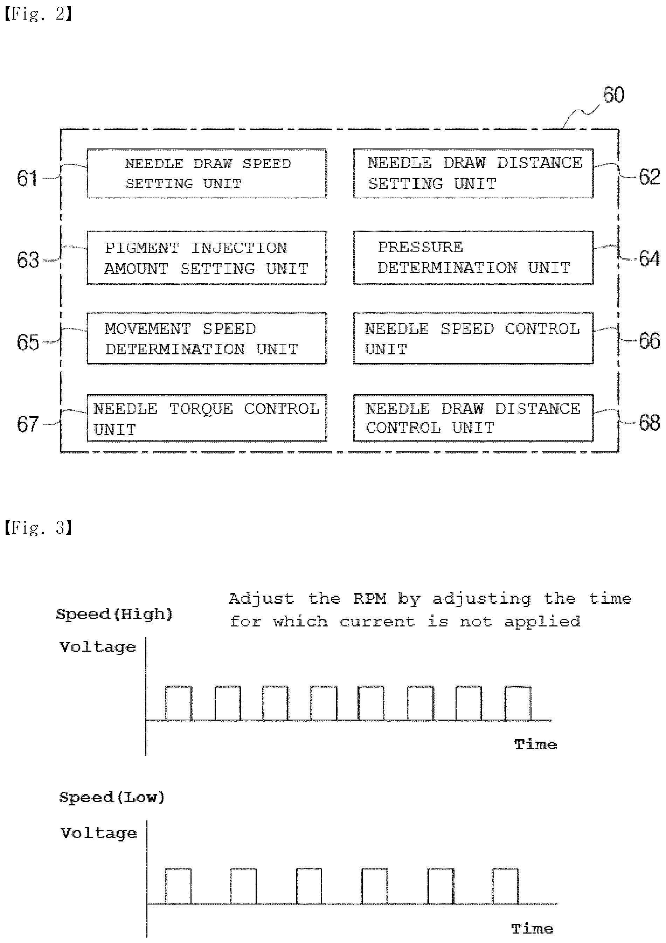

For example, as shown in FIG. 3, the needle speed control unit 66 adjusts the needle driving speed (rpm) by adjusting the interval time, which is the time during which the current of the solenoid is not applied. When the interval time is short as in the upper waveform, the needle driving speed at which the needle is drawn/retracted for the same time is increased. In contrast, when the interval time is longer as in the lower waveform, the needle driving speed at which the needle is drawn/retracted for the same time is decreased. In other words, when the interval time during which no current is applied to the solenoid is shortened, the needle driving speed per time (per second) increases. In contrast, when the interval time is relatively increased, the needle driving speed per time (per second) decreases.

Through this process, the needle speed is controlled.

Meanwhile, the needle speed control unit 66 controls burst stroking by adjusting the ON/OFF driving cycle and interval time of the solenoid according to the burst mode set by the user.

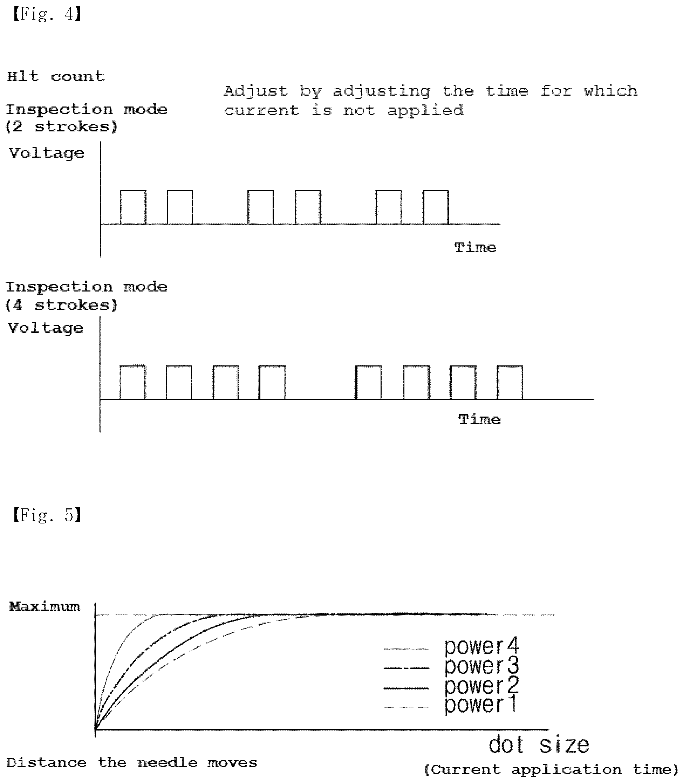

That is, as shown in the upper part of FIG. 4, when a practitioner selects a 2-stroke burst mode, the needle speed control unit 66 controls burst stroking in a cycle of turning on/off the solenoid twice at predetermined time intervals and then having a predetermined time interval, turning on/off the solenoid twice at the predetermined time intervals and then having the predetermined time interval, turning on/off the solenoid twice at the predetermined time intervals and having the predetermined time interval, and so forth.

Furthermore, as shown in the lower part of FIG. 4, when a practitioner selects a 4-stroke burst mode, the needle speed control unit 66 control burst stroking in a cycle of turning on/off the solenoid four times at predetermined time intervals and then having a predetermined time interval, turning on/off the solenoid four times at the predetermined time intervals and then having the predetermined time interval, turning on/off the solenoid four times at the predetermined time intervals and having the predetermined time interval, and so forth.

Although it is actually possible to control the draw speed (rpm) of a needle in the conventional motor-type tattoo device, it is extremely difficult to draw the needle only once and then stop the needle or to draw the needle only twice and then stop the needle. Accordingly, in the conventional motor-type tattoo device, it is impossible to control the burst mode as in the present invention. In contrast, the present invention employs a needle control method using a solenoid, so that the burst mode may be simply implemented by adjusting the driving time of the solenoid.

In addition, the pigment injection amount setting unit 63 extracts a pigment injection amount control value from the memory 80 based on the amount of pigment to be injected set by the setting unit 10 and then sets the amount of pigment to be injected.

In this case, the amount of pigment to be injected may depend on the needle speed. When the needle speed is high, the amount of pigment to be injected may be relatively small. In contrast, when the needle speed is low, the amount of pigment to be injected may be relatively large.

The needle speed control unit 66 may control the needle speed in the same manner as described above while taking into consideration the amount of pigment to be injected.

Meanwhile, as another feature of the present invention, the force (torque) pushing the needle according to the skin condition is controlled separately from the needle speed, and the force of the needle penetrating the skin is adjusted, so that an optimal tattoo can be implemented.

For example, as shown in FIG. 5, the force (torque) pushing the needle is varied regardless of the needle driving speed, it may be possible to achieve optimal tattooing without causing a curve on the skin surface.

In other words, in the conventional motor-type tattoo device, when the needle draw speed is reduced to widen the gap between stroke points, the force is relatively reduced, making it impossible for the needle to pierce the skin. When the needle draw speed is increased to increase the speed of tattooing the speed of the practitioner's hand is also relatively increased, resulting in skin tearing. As described above, although the distance of the needle can be adjusted in the conventional motor-type tattoo device, there is a problem in that the distance of the needle inserted into the skin needs to eventually depend on the skill of the practitioner's hand.

In order to mitigate this problem, in the present invention, only the needle torque is controlled separately from the needle speed, so that pigment is injected into a desired skin layer through force control even when the needle draw speed is low.

That is, as shown in FIG. 5, the needle torque control unit 67 adjusts force by varying the power (voltage) applied to the solenoid. As the power increases, the needle is pushed with stronger force (torque), so that it is possible to shorten the time to reach the maximum distance even when the needle speed is low. This allows the needle to penetrate the skin without causing a curve on the surface of the skin. It is preferable that the depth of the needle is adjusted appropriately by the practitioner according to the condition of the skin.

Although all types of tattooing may be performed using the maximum power of power 4, the overheating time of the handpiece may be shortened when the power is high, so that it may not be suitable for long-term continuous tattooing. It is preferable that a practitioner adjust the depth appropriately after inspecting the condition of the skin. The adjustment of the depth of the needle is a feature that is also possible because the needle driving part is a solenoid.

As the needle moves faster, smaller dots may be marked (the amount of pigment entering the skin is decreased) because the needle is moved with a smaller amount of pigment applied to the surface thereof. When the needle speed is decreased, larger dots may be marked.

The conventional motor-type tattoo device may have a problem in that unwanted large dots are generated because, when the movement speed of a practitioner's wrist is low in the case where a device with a fast needle movement speed is employed, the needle hits a single point a lot. The present invention employs a solenoid method, so that only a desired number of dots may be marked in such a way that the needle speed is decreased and the interval time of the solenoid is increased, with the result that a practitioner can easily adjust the size of dots.

In scalp tattooing, in order to overcome the phenomenon in which the scalp is exposed due to lack of density in the presence of existing hair, it is rather necessary to work with a higher density than bald hair so that the result can be noticed. However, when large dots are marked many times, unnatural results will be obtained due to an excessively dark color. In order to overcome this, it is necessary to perform tattooing while maintaining the color of the scalp to some extent between the dots in such a way as to mark countless dots that are so small that they are not visible to the naked eye. For really small dots, it is necessary to stick the needle into one spot once. Since the dots are smaller, it is necessary to mark a larger number of dots.

This eventually results in a longer tattooing time. In order to overcome this, the present invention may adjust the distance between dots by adjusting the speed of the needle and the movement speed of the hand. The conventional motor-type tattoo device has a fast needle speed, so that a line rather than dots is generated when tattooing is performed as described above. In contrast, in the present invention, the effect of shortening the overall tattooing time may be obtained when dots are marked in lines rather than one by one.

As described above, since the present invention may automatically adjust the depth of a dot and the number of injections, the stress on a practitioner's wrist may be reduced because tattooing time is shortened, and eye fatigue may also be reduced because a practitioner only views a tattooing area, not the tip of the needle. There is provided a tattoo device by which tattooing can be sufficiently performed by not only young practitioners but also older people due to the reduction in physical fatigue.

Meanwhile, as another feature of the present invention, when tattooing starts, the movement speed detection unit 50 detects the movement speed value of the handpiece using an acceleration sensor or the like and transmits the detected movement speed value to the tattoo control unit 60. At the same time, the pressure detection unit 40 also measures the pressure between the scalp and the probe using a pressure sensor or the like and transmits the measured pressure to the tattoo control unit 60 in real time.

The movement speed determination unit 65 of the tattoo control unit 60 compares the movement speed value detected by the movement speed detection unit 50 with the reference movement speed range value registered in the memory 80, and, when an abnormality occurs, generates a speed determination value and transfers it to the needle speed controller 66. That is, when the movement speed value detected by the movement speed detection unit 50 exceeds or falls short of the reference movement speed range, a movement speed determination value is generated and transmitted to the needle speed controller 66. In this case, when the movement speed of the handpiece is excessively fast or excessively slow, a uniform and natural hairstyle may not be implemented. For example, when the moving speed is excessively fast, the amount of pigment injected is small, so that the hair is not appropriately implemented, and thus the effect of the scalp tattoo is poor. When the movement speed is excessively slow, the amount of pigment injected in a specific area may be increased, resulting in staining or a color that is excessively dark compared to the surrounding area. Therefore, it is preferable to perform scalp tattooing while moving the handpiece within a predetermined movement speed range.

When a movement speed determination value is transferred from the movement speed determining unit 65, the needle speed control unit 66 automatically reduces the needle draw speed when the movement time of the hand is out of the set range of the movement time based on the transferred speed determination value, thereby preventing the occurrence of a stain or a change in the density of dots at a specific location. It is also desirable to display a warning message through the display unit 70 as needed. Furthermore, when the movement time of the hand exceeds the set range of the movement time, the needle driving unit is stopped. When the tattoo device suddenly stops during scalp tattooing, a practitioner checks a warning message through the display unit 70 and recognizes that the movement speed of the handpiece is excessively slow or excessively fast. Thereafter, the tattoo device is operated again, and tattooing is performed while paying attention to the movement speed.

In addition, the pressure determination unit 64 of the tattoo control unit 60 compares the pressure value detected by the pressure detection unit 40 with the reference pressure range value registered in the memory 80, and, when an abnormality occurs, generates a pressure determination value and transfers the generated pressure determination value to the needle draw distance control unit 68.

That is, when the pressure value detected by the pressure detection unit 40 exceeds or falls short of the reference pressure range value, a pressure determination value is generated and transferred to the needle draw distance control unit 68. In this case, when the pressure between the probe and the scalp is excessively high or excessively low, it is difficult to inject pigment into an exact scalp layer (a thin layer), making it impossible to implement a uniform and natural hairstyle. For example, pigment is injected into an undesirable deep layer when the pressure value is excessively high and pigment is injected only into an undesirable scalp surface when the pressure value is excessively low, so that a disadvantage arises in that hair is not appropriately implemented. Therefore, it is desirable to perform scalp tattooing within a predetermined pressure range.

In addition, when a movement speed determination value is transferred from the movement speed determination unit 65, the needle draw distance control unit 68 automatically controls the needle draw distance based on the transferred speed determination value. That is, when the pressure value is higher than the predetermined range, the draw distance control value is retrieved from the memory 80 and the solenoid of the needle driving unit 30 is controlled such that the needle draw distance is relatively shortened. When the pressure value is lower than the predetermined range, the withdrawal distance adjustment value is retrieved from the memory 80 and the solenoid of the needle driver 30 is controlled such that the needle draw distance is relatively increased.

As described above, the present invention prevents the pigment injected into the surface of the skin from spreading by individually controlling the torque, which is an instantaneous force, differently from the needle speed. In this case, the force is the force used to penetrate the skin. Generally, the force becomes stronger when the needle speed increases and the force becomes weaker when the needle speed decreases, which is a characteristic of general tattoo devices using a motor. In a situation where the speed is low, the force used to penetrate the scalp is also low, and relatively, the needle is not inserted to the desired thickness of the skin, so that a disadvantage arises in that pigment is injected into the surface of the scalp and spreads.

In order to mitigate the disadvantages of the conventional tattoo devices, the present invention employs the solenoid and individually controls the needle speed and the torque, which is the power used to penetrate the skin. Accordingly, even in a situation where the needle speed is low, the torque is increased such that pigment is always injected at an appropriate depth of the scalp, thereby preventing the pigment from spreading.

In the same manner, in a situation where the needle speed is high, the torque is appropriately decreased such that pigment is always injected at an appropriate depth of the scalp, thereby preventing the pigment from spreading.

Although the invention contrived by the present inventor has been specifically described according to the above embodiments, the present invention is not limited to the above embodiments. It is obvious to those skilled in the art that various changes may be made without departing from the gist of the present invention.

### (Description of Reference Symbols)

10: setting unit
20: needle unit
30: needle drive unit
40: pressure detection unit
50: movement speed detection unit
60: tattoo control unit
61: needle draw speed setting unit
62: needle draw distance setting unit
63: the pigment injection amount setting unit
64: pressure determination unit
65: speed determination unit
66: needle speed control unit
67: needle torque control unit
68: needle draw distance control unit
70: display unit
80: memory
90: power supply unit
100: pigment supply unit
200: handpiece

## Claims

1. A tattoo device comprising:
a tattoo device body including a setting unit configured to set a needle draw distance, a needle draw speed, and an amount of pigment to be injected, and a tattoo control unit configured to control tattoo pigment to be accurately injected into a target skin layer by individually controlling a draw speed of a needle and a needle pushing force according to the needle draw distance, the needle draw speed, and the amount of pigment to be injected set by the setting unit; and
a handpiece coupled to the tattoo device body, and provided with a needle drive unit that controls driving of the needle mounted in a needle unit by using a solenoid under the control of the tattoo control unit.

2. The tattoo device of claim 1, wherein the handpiece comprises:
the needle unit equipped with the needle;
a needle guide configured to guide the needle through drawing thereof, to prevent hair from coming into direct contact with the needle during scalp tattooing, and to fix the skin, which may shake when the needle penetrates the skin, by applying pressure after coming into contact with the skin so that the needle is made to accurately penetrate a set skin layer;
a movement speed detection unit configured to detect a movement speed of the handpiece; and
a pressure detection unit configured to detect a pressure between the skin and the needle unit;
wherein the needle guide includes an elastic member formed of a silicon spring for elastically supporting a needle pin; and
wherein the tattoo control unit controls operations of the tattoo device according to the detected movement speed and the detected pressure value.

3. The tattoo device of claim 1, wherein the tattoo control unit controls the draw speed of the needle by adjusting an interval between operations of the solenoid.

4. The tattoo device of claim 1, wherein the tattoo control unit controls a burst mode by adjusting times of ON/OFF operations of the solenoid.

5. The tattoo device of claim 1, wherein the tattoo control unit adjusts a depth at which the needle penetrates the skin by adjusting the needle pushing force according to the skin.

6. The tattoo device of claim 1, wherein the tattoo control unit includes a needle draw speed setting unit configured to set a needle draw speed based on the needle draw speed set by the setting unit, a needle draw distance setting unit configured to set a needle draw distance based on the needle draw distance set by the setting unit, and a pigment injection amount setting unit configured to set an amount of pigment to be injected based on the amount of pigment to be injected set by the setting unit.

7. The tattoo device of claim 6, wherein the tattoo control unit further includes a needle speed control unit configured to control a needle driving speed according to the set needle draw speed and to control ON/OFF operations of the solenoid based on a detected handpiece movement speed or the set needle draw speed, a needle draw distance control unit configured to variably control a needle draw distance in real time according to the set needle draw distance and a detected pressure value, and a needle torque control unit configured to control a needle pushing torque according to the set amount of pigment to be injected, thereby preventing the pigment from spreading.

8. The tattoo device of claim 7, wherein the needle speed control unit controls a number of dots at one point by adjusting an interval between the ON/OFF operations of the solenoid for drawing the needle, and controls a burst mode by adjusting a number of the ON/ OFF operations of the solenoid per cycle.

9. The tattoo device of claim 7, wherein the needle draw distance control unit controls the draw distance of the needle by adjusting an ON driving time of the solenoid.
